# EUROPÄISCHE PATENTANMELDUNG

(11) **EP 3 257 757 A1**
(43) Veröffentlichungstag der Anmeldung: **20.12.2017**
(21) Anmeldenummer: 16001347.0
(22) Anmeldetag: 15.06.2016
(51) Int. Cl.: B65B 1/16, B65B 1/26, B65B 1/36, B65B 1/44, B65B 1/48, B65B 37/08, B65B 37/20, G01F 11/10, B65B 57/14, G01G 9/00

(54) **DOSIEREINRICHTUNG FÜR PULVER UND VERFAHREN ZUR DOSIERUNG VON PULVER**

(71) Anmelder: Harro Höfliger Verpackungsmaschinen GmbH, 71573 Allmersbach im Tal (DE)
(72) Erfinder: WOLF, Achim, 71573 Allmersbach (DE); KÜHNLE, Julian, 71573 Allmersbach (DE); SAUER, Hartwig, 71573 Allmersbach (DE); HOFMEISTER, Jonas, 71573 Allmersbach (DE)
(74) Vertreter: Zurhorst, Stefan

(57) **Zusammenfassung**

Die Erfindung betrifft eine Dosiereinrichtung (1) zum volumetrischen Dosieren eines insbesondere pharmazeutischen Pulvers (2) und ein zugehöriges Verfahren. Die Dosiereinrichtung (1) umfasst mindestens eine Dosierkavität (10) zur Bildung je einer dosierten Pulvereinzelmenge (30), wobei die Dosierkavität (10) eine offene Seite (14) und gegenüberliegend dazu einen Boden aufweist. Der Boden der jeweiligen Dosierkavität (10) ist zumindest abschnittsweise durch ein luftdurchlässiges Filterelement (15) gebildet. Die Dosiereinrichtung (1) weist einen Druckkanal (16) auf, der mit der mindestens einen Dosierkavität (10) durch das Filterelement (15) hindurch druckübertragend verbunden ist. Auf der der Dosierkavität (10) abgewandten Seite des Filterelementes (15) ist eine Prüflichtquelle (31) angeordnet. Das Filterelement (15) ist bezüglich des von der Prüflichtquelle (31) ausgehenden Lichtes durchscheinend ausgestaltet. Die Dosiereinrichtung (1) umfasst ferner eine Prüfkamera (32) sowie eine mit der Prüfkamera (32) verbundene Auswerteeinrichtung (33). In der Auswerteeinrichtung (33) werden aus der von der Prüfkamera (32) aufgenommenen Lichtintensität des von der Prüflichtquelle (31) ausgehenden und durch das Filterelement (15) scheinenden Lichtes Rückschlüsse auf den Füllzustand der Dosierkavität (10) gezogen.

## Beschreibung

Die Erfindung betrifft eine Dosiereinrichtung zum volumetrischen Dosieren eines insbesondere pharmazeutischen Pulvers der im Oberbegriff des Anspruchs 1 angegebenen Gattung sowie ein Verfahren zur Dosierung eines solchen Pulvers mittels der genannten Dosiereinrichtung.

Für die ordnungsgemäße Verwendung von beispielsweise pharmazeutischem Pulver durch die Zielperson müssen exakt dosierte Teilmengen beispielsweise in Kapseln, in Blisterverpackungen, in sogenannten DPI-Discs (Dry Powder Inhaler Disc) oder anderen Zielkavitäten bereitgestellt werden. Verbreitet findet hierfür zunächst eine volumetrische Dosierung des Pulvers statt. Hierfür weist die zugehörige Dosiereinrichtung eine oder mehrere Dosierkavitäten auf, die mit Pulver befüllt werden. Bei ordnungsgemäßer Befüllung definiert das Volumen der Dosierkavitäten jeweils eine volumetrisch dosierte Pulvereinzelmenge. Solchermaßen dosierte Pulvereinzelmengen werden in die zugehörigen Zielkavitäten eingefüllt.

Die Dosiereinrichtung ist typischerweise derart aufgebaut, dass die mindestens eine Dosierkavität eine offene Seite und gegenüberliegend dazu einen Boden aufweist. Der Boden der jeweiligen Dosierkavität ist zumindest abschnittsweise durch ein luftdurchlässiges Filterelement gebildet. Außerdem weist die Dosiereinrichtung einen Druckkanal auf, der mit der mindestens einen Dosierkavität durch das Filterelement hindurch druckübertragend verbunden ist. Die zuvor volumetrisch abgemessene Pulvereinzelmenge wird mittels durch den Druckkanal und durch das Filterelement hindurch aufgebrachten Gas- bzw. Luftdruck aus der Dosierkavität in die Zielkavität hinein geblasen. Außerdem ist es möglich, durch den Druckkanal und durch das Filterelement hindurch die Dosierkavität mit Unterdruck zu beaufschlagen. Hierdurch kann frisches Pulver in die Dosierkavität eingesaugt und bis zur Überführung in die Zielkavität gehalten werden.

Für die Erzielung eines wiederholbar genauen Dosierergebnisses muss sichergestellt werden, dass einerseits der Füllgrad mit Pulverdichte bzw. Dichteverteilung in den Dosierkavitäten wiederholbar beherrscht werden kann, und dass andererseits der vollständige Austrag der dosierten Pulvereinzelmengen aus den Dosierkavitäten in die Zielkavitäten erreichbar ist. Unterschiedliche Pulverarten haben jedoch sehr unterschiedliche Verarbeitungseigenschaften. Insbesondere mehr oder weniger ausgeprägte Neigungen des Pulvers zum Haften an Oberflächen und zur Agglomeratbildung stehen einer gleichmäßigen Füllung der Dosierkavitäten und einer zuverlässigen Übergabe der abgemessenen Pulvermengen in die Zielkavitäten entgegen.

Der Erfindung liegt die Aufgabe zugrunde, eine Dosiereinrichtung zum volumetrischen Dosieren eines insbesondere pharmazeutischen Pulvers mit verbesserter Wiederholgenauigkeit anzugeben.

Diese Aufgabe wird durch eine Dosiereinrichtung mit den Merkmalen des Anspruchs 1 gelöst.

Der Erfindung liegt des Weiteren die Aufgabe zugrunde, ein Dosier- und Füllverfahren anzugeben, mittels dessen mit erhöhter Genauigkeit und Zuverlässigkeit bei hohen Taktraten Einzelmengen von Pulver dosiert und in Zielkavitäten eingefüllt werden können.

Diese Aufgabe wird durch ein Verfahren mit den Merkmalen des Anspruchs 6 gelöst.

Nach der Erfindung ist vorgesehen, dass auf der der Dosierkavität abgewandten Seite des Filterelementes eine Prüflichtquelle angeordnet ist, und dass das Filterelement bezüglich des von der Prüflichtquelle ausgehenden Lichtes durchscheinend ausgestaltet ist. Ferner umfasst die Dosiereinrichtung eine Prüfkamera sowie eine mit der Prüfkamera verbundene Auswerteeinrichtung, wobei die Prüfkamera zumindest für einen Erfassungszeitraum auf die offene Seite der Dosierkavität gerichtet ist, und wobei die Auswerteeinrichtung (33) dazu ausgelegt ist, aus der von der Prüfkamera aufgenommenen Lichtintensität des von der Prüflichtquelle ausgehenden und durch das Filterelement scheinenden Lichtes Rückschlüsse auf den Füllzustand der Dosierkavität zu ziehen.

Das zugehörige erfindungsgemäße Verfahren umfasst folgende Verfahrensschritte: Pulver wird in die Dosierkavität eingefüllt und später mittels durch den Druckkanal und durch das Filterelement hindurch aufgebrachten Gasdruck aus der Dosierkavität ausgeblasen. Die Dosierkavität wird mittels der Prüflichtquelle durch das Filterelement hindurch durchleuchtet. Die Prüfkamera wird zumindest für einen Erfassungszeitraum auf die offene Seite der Dosierkavität gerichtet. Die Lichtintensität des von der Prüflichtquelle ausgehenden und durch das Filterelement scheinenden Lichtes wird von der Prüfkamera aufgenommen und in der Auswerteeinrichtung ausgewertet. In der Auswerteeinrichtung werden aus der von der Prüfkamera aufgenommenen Lichtintensität Rückschlüsse auf den Füllzustand der Dosierkavität gezogen.

Es hat sich gezeigt, dass sich eine zumindest ausreichende, im Regelfall sogar sehr gute Korrelation zwischen der aufgenommenen Lichtintensität und der in der jeweiligen Dosierkavität befindlichen Pulvermenge herstellen lässt, und dass dieser Umstand für eine In-Prozess-Kontrolle genutzt werden kann. In einem Kontrollschritt wird die jeweilige Dosierkavität von innen, also durch das ohnehin vorhandene Filterelement hindurch durchleuchtet. Die hindurchtretende Lichtmenge wird durch in der Dosierkavität enthaltenes Pulver reduziert. Durch Vergleich des mittels der Prüfkamera tatsächlich aufgefundenen Lichtsignals mit einem Referenzlichtsignal können in der entsprechend ausgelegten Auswerteeinrichtung Rückschlüsse auf den Füllzustand der Dosierkavität gezogen und bei Bedarf entsprechende Maßnahmen eingeleitet werden. In jedem Fall wird davon ausgegangen, dass das durch das Filterelement hindurchtretende, ansonsten aber ungestörte Licht eine bestimmte kalibrierte Intensität bzw. Intensitätsverteilung hat, und dass eine in der Dosierkavität befindliche Pulvermenge zu einer Abschwächung der zuvor erwähnten ungestörten, kalibrierten Lichtmenge führt. Im einfachsten Fall wird nur eine ja/nein-Prüfung dahingehend vorgenommen, ob sich überhaupt Pulver in der jeweiligen Dosierkavität befindet oder nicht. Sofern das aufgenommene Lichtsignal schwächer ist als das Referenzlichtsignal einer leeren Dosierkavität, wird dies als Signal für das Vorhandensein von Pulver in der Dosierkavität gewertet, wofür eine einfache hell/dunkel-Erkennung ausreichen kann. Im Regelfall ist jedoch auch die in der Dosierkavität vorhandene, dosierte Pulvereinzelmenge in einem bestimmten Grad durchscheinend bzw. lichtdurchlässig, wobei der Grad der Lichtdurchlässigkeit von der Menge bzw. von der Dichte des Pulvers in der Dosierkavität abhängt. Nach einer geeigneten Kalibrierung kann durch Auswertung der insgesamt durchgelassenen und aufgenommenen Lichtintensität nicht nur festgestellt werden, ob überhaupt Pulver vorhanden ist, sondern es kann auch eine Aussage über die Menge des in der Dosierkavität befindlichen Pulvers getroffen werden. Schließlich besteht die genaueste und im Rahmen der Erfindung auch bevorzugte Methode darin, dass mittels der der Prüfkamera eine Verteilung der Lichtintensität über der Fläche der offenen Seite einer Dosierkavität aufgenommen wird, und dass mittels einer Bildanalyse der aufgenommenen Verteilung der Lichtintensität Rückschlüsse auf den Füllzustand der Dosierkavität gezogen werden. Diese Weiterbildung des erfindungsgemäßen Verfahrens basiert auf der Erkenntnis, dass sich innerhalb einer einzelnen Dosierkavität selbst bei ordnungsgemäßer Befüllung niemals eine exakt homogene Dichteverteilung des Pulvers einstellt. Durch die Bildanalyse werden relativ helle und relativ dunkle Bildabschnitte als Zonen mit relativ geringer bzw. relativ hoher Pulverdichte erkannt, wobei nach geeigneter Kalibrierung das Maß der aufgenommenen Helligkeit als Maß für die örtliche Pulverdichte herangezogen werden kann. Durch Integration der Helligkeitsverteilung kann die für das Dosierergebnis relevante, in der Dosierkavität befindliche Pulvergesamtmenge bestimmt werden.

In einer bevorzugten Ausgestaltung des erfindungsgemäßen Verfahrens findet die oben beschriebene Prüfung nach dem Prozessschritt der Einfüllung des Pulvers in die Dosierkavität, jedoch vor dem Prozessschritt der Übergabe des Pulvers aus der Dosierkavität in die Zielkavität statt. Hierdurch wird erkannt, ob überhaupt eine ordnungsgemäße Befüllung der Dosierkavität stattgefunden hat. Abhängig vom Prüfergebnis, insbesondere bei einem negativen Prüfergebnis, können geeignete Maßnahmen ergriffen werden. Beispielsweise kann eine Nachbefüllung versucht werden. Alternativ kann auf den Prozessschritt der Übergabe des Pulvers in die Zielkavität verzichtet werden. Eine weitere Möglichkeit besteht darin, die Übergabe des Pulvers in die Zielkavität zwar durchzuführen, anschließend aber die dann nur unzureichend befüllten Zielkavitäten auszusondern.

Ergänzend oder als alternative findet die oben beschriebene Prüfung nach dem Prozessschritt der Übergabe des Pulvers aus der Dosierkavität in die Zielkavität statt. Hierdurch wird erkannt, ob die in der Dosierkavität abgemessene Pulvereinzelmenge vollständig an die Zielkavität übergeben wurde, oder ob Restmengen des Pulvers in der Dosierkavität haften geblieben sind. Im letzteren Fall muss davon ausgegangen werden, dass trotz ordnungsgemäßer Befüllung der Dosierkavität keine vollständige, also keine ordnungsgemäße Pulvereinzelmenge in die Zielkavität gelangt ist. Es können dann geeignete Maßnahmen wie zuvor schon beschrieben ergriffen werden.

Die Durchleuchtung von Filter und Pulver kann mit Dauerlicht erfolgen. Für eine gute Bildaufnahme und Erkennbarkeit des Füllgrades hat sich jedoch bewährt, dass innerhalb eines Aufnahmeschrittes mehrere Lichtblitze von der Prüflichtquelle ausgesandt werden. Für den gleichen Zweck ist die Prüflichtquelle bevorzugt in dem an das Filterelement angrenzenden Mündungsbereich des Druckkanals positioniert, wobei sie zweckmäßig Lichtstreumittel für eine gleichmäßige Ausleuchtung des Filterelementes aufweist.

Sofern die Dosiereinrichtung mehrere, gleichzeitig befüllte bzw. gleichzeitig entleerte Dosierkavitäten aufweist, kann es zweckmäßig sein, für jede dieser Dosierkavitätenje eine Prüfkamera vorzusehen. In bevorzugter Weiterbildung sind jedoch die offenen Seiten von mehreren und insbesondere von allen Dosierkavitäten mittels der gleichen, nur einen Prüfkamera gemeinsam erfasst. Über eine softwaremäßige Bildauswertung lassen sich die Befüllungszustände der sämtlich gemeinsam erfassten Dosierkavitäten zuverlässig ermitteln, während der Hardware-Aufwand für die Bilderfassung auf ein Minimum reduziert ist.

Dosiereinrichtung und Verfahren gemäß der Erfindung lassen sich bei sämtlichen Formen der volumetrischen Dosierung einsetzen. Bevorzugt umfasst aber die Dosiereinrichtung eine Dosierstation, eine Füllstation, mindestens eine Prüfstation sowie ein bewegliches Dosierorgan. Dabei sind die Dosierkavität und die Prüflichtquelle im Dosierorgan angeordnet, wobei die Prüfkamera in der mindestens einen Prüfstation positioniert ist. Das Dosierorgan ist zyklisch zwischen der Dosierstation, der Füllstation und der mindestens einen Prüfstation hin und her verfahrbar. Hierdurch lassen sich hohe Taktraten bei Dosierung des Pulvers und Einfüllung der dosierten Pulvereinzelmengen in die Zielbehälter erzielen, wobei die erfindungsgemäße Füllprüfung die Taktrate nicht oder nicht nennenswert bremst. Gleichzeitig sind Wiederholgenauigkeit und Zuverlässigkeit des Gesamtprozesses und damit die Wirtschaftlichkeit gesteigert.

Ausführungsbeispiele der Erfindung sind nachfolgend anhand der Zeichnung näher beschrieben. Es zeigen:
- Fig. 1: in einer schematischen Übersichtsdarstellung ein Ausführungsbeispiel einer erfindungsgemäßen Dosiereinrichtung mit einer Dosierstation, mit einer Füllstation, mit zwei Prüfstationen, und mit Dosierorganen, welche zyklisch von Station zu Station verfahrbar sind;
- Fig. 2: in einer geschnittenen Frontansicht die Anordnung nach Fig. 1 mit Einzelheiten zur Ausgestaltung der zu einer gemeinsam beweglichen Einheit zusammengefassten Dosierorgane in Relation zu Prüfkameras, wobei in den Dosierorganen eine Prüflichtquelle angeordnet ist;
- Fig. 3: in einer vergrößerten Detailansicht ein Dosierorgan nach Fig. 2 im Bereich einer Prüfstation mit Einzelheiten zur Ausgestaltung der Dosierkavitäten mit Filterelementen als Boden und mit einer dahinter positionierten Prüflichtquelle.

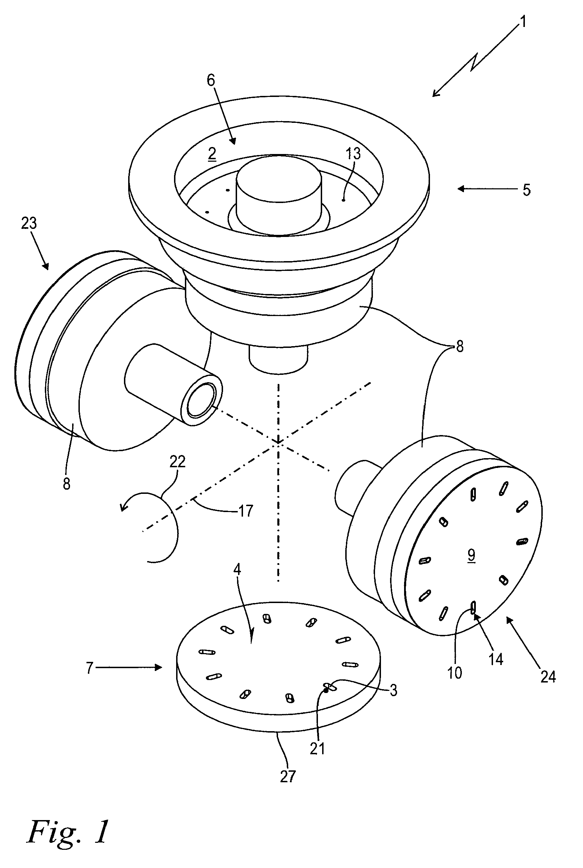

Fig. 1 zeigt in einer schematischen Übersichtsdarstellung ein erstes Ausführungsbeispiel eines erfindungsgemäßen Dosiersystems, welches eine Dosiereinrichtung 1 sowie eine zugeordnete Anzahl von in einer gemeinsamen Ebene 4 angeordneten Zielkavitäten 3 umfasst. Die mehreren Zielkavitäten 3 liegen demnach nicht auf einer gemeinsamen Linie, sondern spannen die Ebene 4 in zwei verschiedenen horizontalen Raumrichtungen auf, so dass also die Ebene 4 horizontal bzw. quer zur Gewichtskraftrichtung ausgerichtet ist. Ferner läuft um die anfänglich einseitig offenen Zielkavitäten 3 eine Siegelfläche 21 um. Auf die Siegelfläche 21 wird nach Befüllung mit einem Pulver 2 eine nicht dargestellte Deckfolie aufgesiegelt, wodurch die befüllten Zielkavitäten 3 dicht verschlossen werden.

Die hier zehn Zielkavitäten 3 sind beispielhaft ringförmig in einer ebenen DPI-Disc 27 (Dry Powder Inhaler Disc) ausgebildet. Eine solche DPI-Disc 27 wird in Inhalationsgeräten zur pulmonalen Verabreichung von pharmazeutischem Pulver 2 eingesetzt. DPI-Discs weisen typischerweise zehn bis einundsechzig Zielkavitäten 3 auf. Anstelle der gezeigten ringförmigen Anordnung der Zielkavitäten 3 ist auch jede beliebige andere geometrische Anordnung beispielsweise in Rechteck-, Polygon- oder Matrixform denkbar, solange die Zielkavitäten 3 in einer gemeinsamen Ebene 4 liegen. Außerdem ist es nicht zwingend erforderlich, dass die Zielkavitäten 3 in einem gemeinsamen Bauteil, hier in der DPI-Disc 27 ausgeformt sind. Es sind auch Zielkavitäten 3 als separate Behälter-oder in Bauteilen zusammengefasste Gruppen davon denkbar. Im Rahmen der Erfindung lässt sich auch eine beliebige andere Anzahl von Zielkavitäten 3 gleichzeitig dosiert befüllen, was auch die Befüllung einer einzelnen Zielkavität 3 einschließt. An Stelle der hier gezeigten Dosiereinrichtung 1 mit mehreren, weiter unten noch näher beschriebenen umlaufenden Dosierorganen 8 können auch Walzendosierer, Stechheber oder nahezu beliebige andere Bauformen von volumetrisch arbeitenden Dosiereinrichtungen im Rahmen der Erfindung angepasst werden und zum Einsatz kommen, was auch die Möglichkeit der Befüllung von in beliebig anderer Weise positionierten Zielbehältern einschließt.

Die Zielkavitäten 3 weisen hier beispielhaft einen länglichen, ovalen Grundriss auf. Jeder andere Grundriss der Zielkavität 3 ist ebenso im Rahmen der Erfindung einsetzbar. Außerdem ist die Erfindung nicht auf die Dosierung von pharmazeutischem Pulver zur pulmonalen Verabreichung beschränkt. Ebenso ist die Erfindung auf eine Dosierung von anderen pharmazeutischen Pulvern oder pulvrigen Nahrungsergänzungsmitteln anwendbar, wobei der hier gewählte Begriff des Pulvers 2 auch Granulate oder dergleichen umfasst.

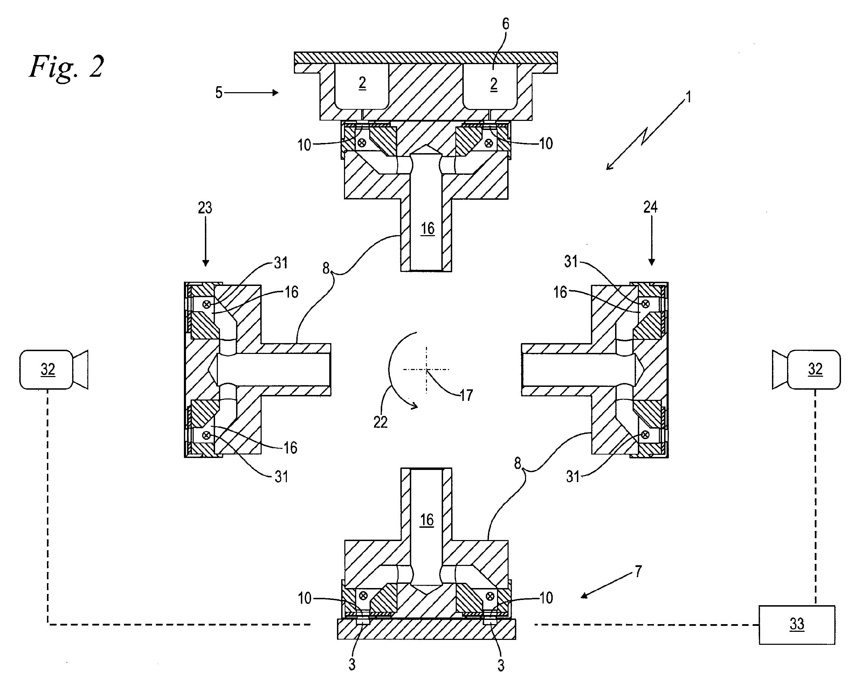

Die Dosiereinrichtung 1 umfasst eine Dosierstation 5 mit einem Pulvervorratsbehälter 6, in dem eine Vorratsmenge von Pulver 2 bereitgehalten wird. Des Weiteren umfasst die Dosiereinrichtung 1 noch eine Füllstation 7 sowie mindestens ein bewegliches Dosierorgan 8. Im gezeigten Ausführungsbeispiel sind entsprechend der Darstellung nach den Fig. 1, 2 insgesamt vier Dosierorgane 8 vorgesehen, von denen das untere Dosierorgan 8 zur besseren Darstellung der DPI-Disc 27 nur in Fig. 2, nicht aber in Fig. 1 gezeigt ist. Die Füllstation 7 ist bezogen auf die Gewichtskraftrichtung unterhalb der Dosierstation 5 angeordnet. Außerdem sind noch mindestens eine oder mehrere, hier zwei Zwischenstationen vorgesehen, deren Funktion als Prüfstation 23, 24 weiter unten näher beschrieben wird. Das mindestens eine Dosierorgan 8 ist zyklisch von der Dosierstation 5 zur Füllstation 7 und wieder zurück verfahrbar, was durch einen Linearhub oder dergleichen realisiert werden kann. Im gezeigten bevorzugten Ausführungsbeispiel ist das

Dosierorgan 8 um eine Rotationsachse 17 schwenkbar, wobei das Dosierorgan 8 in einer oberen 0°-Rotationsposition unterhalb der Dosierstation 5 liegt, und wobei das Dosierorgan 8 in einer unteren 180°-Rotationsposition oberhalb der Füllstation 7 liegt. In entsprechenden Winkel-Zwischenschritten werden mindestens eine, hier zwei weitere Prüfstationen 23, 24 angefahren. Im gezeigten Ausführungsbeispiel sind mehrere, hier beispielhaft vier Dosierorgane 8 um die Rotationsachse 17 herum angeordnet und für ein sequenzielles Anfahren der Dosierstation 5, der Füllstation 7 sowie der Prüfstationen 23, 24 zu einer gemeinsam um die Rotationsachse 17 rotierbaren Einheit zusammengefasst.

Der Darstellung nach Fig. 1 ist noch entnehmbar, dass die Dosierorgane 8 jeweils eine ebene Transferfläche 9 aufweisen, in der korrespondierend zur Anzahl und zur geometrischen Verteilung der Zielkavitäten 3 mehrere Dosierkavitäten 10 eingearbeitet sind. In der Ebene der Transferfläche 9 weisen die Dosierkavitäten 10 jeweils eine vom Grundkörper des Dosierorgans 8 fort weisende offene Seite 14 auf. Bevorzugt, aber nicht zwingend weisen die Dosierkavitäten 10 im Bereich ihrer offenen Seite 14 den gleichen Grundriss wie die Zielkavitäten 3 auf. "Korrespondierend zur Anzahl und zur geometrischen Verteilung bzw. Anordnung" bedeutet hier, dass je eine Dosierkavität 10 an der Füllstation 7 mit je einer Zielkavität 3 in Überdeckung gebracht werden kann. Hierzu sind die Dosierkavitäten 10 ebenso wie die Zielkavitäten 3 ringförmig mit gleicher Winkelteilung und mit gleichem Radius in der Transferfläche 34 ausgebildet. Analoges gilt bei einer abweichenden Verteilung der Zielkavitäten 3 auf der Ebene 4. Obiges bedeutet aber nicht, dass notwendig die gleiche Anzahl von Dosierkavitäten 10 und Zielkavitäten 3 vorhanden sein muss. Es kann auch zweckmäßig sein, dass ausgehend von der Anzahl der Zielkavitäten 3 ein n-facher Teiler davon (1/2, 1/3 oder dgl.) als Anzahl von Dosierkavitäten 10 vorgesehen ist. In diesem Fall können in einem ersten Takt ein erster Teil der Zielkavitäten 3 und in einem oder mehreren nachfolgenden Takten die übrigen Zielkavitäten 3 befüllt werden.

Weiter ist der Darstellung von Fig. 1 noch entnehmbar, dass im Boden des Pulvervorratsbehälters 6 eine Anzahl von Pulverkanälen 13 ausgebildet ist. Jeder Dosierkavität 10 ist mindestens je ein Pulverkanal, hier genau ein Pulverkanal 13 zugeordnet. Es kann aber auch zweckmäßig sein, dass jeder Dosierkavität 10 zwei oder mehr Pulverkanäle 13 zugeordnet sind. Das im Pulvervorratsbehälter 6 befindliche Pulver 2 wird durch die Pulverkanäle 13 in die jeweiligen Dosierkavitäten 13 geleitet.

Fig. 2 zeigt in einer geschnittenen Frontansicht die Anordnung nach Fig. 1 mit Einzelheiten zur Ausgestaltung der Dosierstation 5 und der zu einer gemeinsam beweglichen, hier gemeinsam um die Rotationsachse 17 schwenkbaren Einheit zusammengefassten Dosierorgane 8. Entsprechend der ringförmigen Anordnung der Dosierkavitäten 10 ist auch der Pulvervorratsbehälter 6 ringförmig ausgebildet, wobei bei der Platzierung des Dosierorgans 8 unterhalb der Dosierstation 5 die ringförmig angeordneten Dosierkavitäten 10 unterhalb des ebenfalls ringförmig ausgebildeten Pulvervorratsbehälters 6 zu liegen kommen. Bei einer abweichenden Anordnung der Dosierkavitäten 10 ist eine analoge Ausgestaltung des Pulvervorratsbehälters 6 zweckmäßig. Sobald das jeweilige Dosierorgan 8 infolge der Schwenkbewegung um die Rotationsachse 17 entsprechend dem Pfeil 22 die untere Füllstation 7 erreicht hat, liegen die Dosierkavitäten 10 exakt über je einer zugeordneten Zielkavität 3. Weitere Einzelheiten zur Zusammenschau der Fig. 1 und 2 sind weiter unten noch beschrieben.

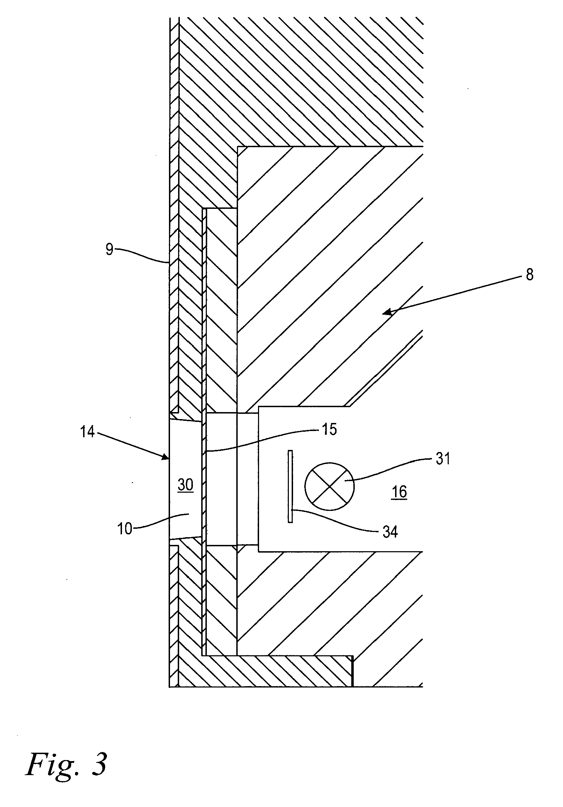

Fig. 3 zeigt in einer vergrößerten Detailansicht dasjenige Dosierorgan 8 nach den Fig. 1, 2, welches sich im Bereich der Prüfstation 23 befindet. Weiter oben wurde schon erwähnt, dass die Dosieröffnungen 12 außen bzw. in der 0°-Rotationsposition nach Fig. 1 oben jeweils eine offene Seite 14 aufweisen. Gegenüberliegend dazu, also hier unten bzw. bezogen auf die senkrecht zur Rotationsachse 17 (Fig. 1) liegende Radialrichtung innen, sind die Dosierkavitäten 10 durch einen Boden begrenzt. Der Boden der Zielkavitäten 10 ist zumindest abschnittsweise, hier vollflächig durch ein luftdurchlässiges Filterelement 15 ausgebildet. Das Filterelement 15 ist bezüglich seiner Durchlässigkeit auf das Pulver 2 derart abgestimmt, dass zwar Luft, nicht aber Pulverpartikel hindurchtreten können. Außerdem ist der Mündungsbereich eines Druckkanals 16 erkennbar, der mit der mindestens einen Dosierkavität 10 durch das Filterelement 15 hindurch druckübertragend verbunden ist. Auf der der Dosierkavität 10 abgewandten Seite des Filterelementes 15 ist eine Prüflichtquelle 31 angeordnet. Außerdem weist die Prüflichtquelle 31 optionale Lichtstreumittel 34 für eine gleichmäßige Ausleuchtung des Filterelementes 15 auf. Das Filterelement 15 ist bezüglich des von der Prüflichtquelle 31 ausgehenden Lichtes durchscheinend ausgestaltet.

Unter erneutem Bezug auf Fig. 2 ist noch erkennbar, dass in jedem Dosierorgan 8 je ein zentraler Druckkanal 16 ausgebildet ist, der über entsprechende Verzweigungen mit allen Dosierkavitäten 10 durch die Filterelemente 15 hindurch luftdruckübertragend verbunden ist. In seinem an die Dosierkavitäten 10 bzw. deren Filterelemente 15 angrenzenden Mündungsbereich ist der Druckkanal 16 korrespondierend zu den ringförmig positionierten Dosierkavitäten 10 ebenfalls ringförmig ausgebildet. Die weiter oben schon im Zusammenhang mit Fig. 3 erwähnte Prüflichtquelle 31 ist hier im genannten Mündungsbereich des Druckkanals 16 positioniert. Es kann zweckmäßig sein, für jedes Filterelement 15 je eine individuelle Prüflichtquelle 31 vorzusehen. Im gezeigten bevorzugten Ausführungsbeispiel ist die Prüflichtquelle 31 als ringförmig im Mündungsbereich des Druckkanals 16 umlaufendes Lichtband mit LED-Lichtquellen ausgebildet.

Außerdem ist in Fig. 2 noch erkennbar, dass die Dosiereinrichtung 1 mindestens eine Prüfkamera 32 sowie eine mit der mindestens einen Prüfkamera 32 verbundene Auswerteeinrichtung 33 umfasst. Die Prüfkamera 32 ist zumindest für einen Erfassungszeitraum auf die offene Seite 14 der Dosierkavität 10 gerichtet. Im gezeigten Ausführungsbeispiel sind hierzu mindestens eine, hier zwei Zwischenstationen vorgesehen, die als Prüfstationen 23, 24 zum Einsatz kommen und mit je einer Prüfkamera 32 versehen sind. Innerhalb einer Prüfstation 23, 24 sind die offenen Seiten 14 von mehreren, hier von allen Dosierkavitäten 15 des jeweils dort positionierten Dosierorgans 8 mittels der jeweiligen Prüfkamera 32 gemeinsam erfasst. Die Prüfkameras 32 sind derart eingerichtet, dass sie in den Prüfstationen 23, 24 das von der Prüflichtquelle 31 ausgehende, durch das Filterelement 15 scheinende und aus der offenen Seite 14 austretende Licht aufnehmen. Die Auswerteeinrichtung 33 ist dazu ausgelegt und eingerichtet, aus der von der Prüfkamera 32 aufgenommenen Lichtintensität des von der Prüflichtquelle 31 ausgehenden und durch das Filterelement 15 scheinenden Lichtes gemäß unten stehender Beschreibung Rückschlüsse auf den Füllzustand der Dosierkavität 10 zu ziehen.

Ausgehend von Fig. 3 und unter gleichzeitigem Bezug auf die Darstellung nach den Fig. 1 und 2 wird folgendes erfindungsgemäße Verfahren ausgeführt:
Zunächst wird ein Dosierorgan 8 zur Dosierstation 5 verfahren, welche sich in der Darstellung nach Fig. 2 in der oberen 0°-Position bzw. in der 12-Uhr-Position befindet. In dieser Position wird nun durch den zentralen Druckkanal 16, durch die Filterelemente 15, die Dosierkavitäten 10 und die Pulverkanäle 13 hindurch eine negative Druckdifferenz auf das Pulver 2 im Pulvervorratsbehälter 6 aufgebracht. Der hier gewählte Begriff der "negativen Druckdifferenz" bedeutet zunächst allgemein, dass im Druckkanal 16 ein geringerer Luftdruck herrscht als außenseitig des Pulvervorrats. Umgekehrt bedeutet eine "positive Druckdifferenz", dass im Druckkanal 16 ein höherer Druck als im Pulvervorratsbehälter 6 herrscht. Eine negative Druckdifferenz kann beispielsweise dadurch herbeigeführt werden, dass der Pulvervorratsbehälter 6 geschlossen und innen mit einem Überdruck beaufschlagt wird, während im Druckkanal 16 relativ dazu ein geringerer Druck, beispielsweise atmosphärischer Umgebungsdruck herrscht. Bevorzugt herrscht im Pulvervorratsbehälter 6 atmosphärischer Umgebungsdruck, während mittels einer nicht dargestellten Unterdruckquelle (Vakuumpumpe) ein Unterdruck im Druckkanal 16 ausgebildet wird. In jedem Falle führt die negative Druckdifferenz dazu, dass das im Pulvervorratsbehälter 6 bereitgestellte Pulver durch die Pulverkanäle 13 in die Dosierkavitäten 10 gesaugt und dort an den Filterelementen 15 zurückgehalten wird, während mitgeführte Luft und die zuvor in den Dosierkavitäten 10 befindliche Luft durch die Filterelemente 15 und den Druckkanal 16 entweicht bzw. abgesaugt wird.

Hierdurch werden alle Dosierkavitäten 10 eines einzelnen Dosierorgans 8 gleichzeitig vollständig mit Pulver 2 befüllt und dabei infolge des definierten Volumens der einzelnen Dosierkavitäten 10 volumetrisch exakt zu Pulvereinzelmenge 30 des Pulvers 2 dosiert.

Es kann zweckmäßig sein, während der Befüllung der Dosierkavitäten 10 eine kontinuierliche, gleichbleibende negative Druckdifferenz aufrechtzuerhalten. Bevorzugt wird mindestens eine impulsartige negative Druckdifferenz aufgebracht. Ebenso kann es zweckmäßig sein, einen oder mehrere negative Druckimpulse mit einer kontinuierlichen negativen Grunddruckdifferenz zu kombinieren.

Im Anschluss an die Befüllung der Dosierkavitäten 10 wird erneut eine negative Druckdifferenz aufgebracht. Unter Aufrechterhaltung dieser negativen Druckdifferenz wird das Dosierorgan 8 von der Dosierstation 5 zur ersten Prüfstation 23 verfahren, wie es in Fig. 2 erkennbar ist. Die Aufrechterhaltung der negativen Druckdifferenz verhindert während der dazwischen liegenden Rotationsbewegung, dass das in den Dosierkavitäten 10 befindliche Pulver 2 aus den offenen Seiten 14 herausfällt.

Beim Erreichen der ersten Prüfstation 23 bzw. der 90°-Rotationsposition oder 9-Uhr-Position entsprechend der Darstellung nach Fig. 2 weisen die Transferfläche 9 des Dosierorgans 8 und die in gleicher Ebene liegenden offenen Seiten 14 der Dosierkavitäten 10 zur Seite, wobei die Dosierkavitäten 10 der dort positionierten Prüfkamera 32 zugewandt zu liegen kommen. Für zumindest einen ersten Erfassungszeitraum ist eine der beiden Prüfkameras 3 2 auf die offenen Seiten 14 der Dosierkavitäten 10 des hier positionierten Dosierorgans 8 gerichtet. Die Filterelemente 15 und das gegebenenfalls darin befindliche Pulver 2 werden hierbei mittels der oben beschriebenen Prüflichtquelle 31 durchleuchtet. Das durchscheinende und aus der offenen Seite 14 austretende Licht wird von der Prüfkamera aufgenommen und in der Auswerteeinrichtung 33 ausgewertet. In der Auswerteeinrichtung 33 werden aus der von der Prüfkamera 32 aufgenommenen Lichtintensität Rückschlüsse auf den Füllzustand der Dosierkavität 10 gezogen. Genauer wird mittels der Prüfkamera 32 eine Verteilung der Lichtintensität über der Fläche der offenen Seite 14 der Dosierkavität 10 aufgenommen. In der Auswerteeinrichtung 33 werden mittels einer Bildanalyse aus der aufgenommenen Verteilung der Lichtintensität Rückschlüsse auf den Füllzustand der Dosierkavität 10 gezogen. Insbesondere kann erkannt werden, ob die einzelnen Dosierkavitäten 10 zuvor in der Dosierstation 5 ordnungsgemäß mit Pulver 2 befüllt wurden, bzw. ob sich in den einzelnen Dosierkavitäten 10 tatsächlich die gewünschte dosierte Pulvereinzelmenge 30 des Pulvers 2 befindet. Falls diese Erkennung zu einem negativen Ergebnis führt, können geeignete Maßnahmen eingeleitet werden.

Anschließend erfolgt eine weitere 90°-Drehung der vorgenannten Anordnung. Auch bei dieser Drehbewegung kann die Aufrechterhaltung der oben beschriebenen negativen Druckdifferenz dazu genutzt werden, dass Herausfallen des in den Dosierkavitäten 10 befindliche Pulver 2 aus den offenen Seiten 14 zu verhindern. Beim Erreichen der Füllstation 7 bzw. der 180°-Rotationsposition oder 6-Uhr-Position entsprechend der Darstellung nach Fig. 2 weisen die Transferfläche 9 des Dosierorgans 8 und die in gleicher Ebene liegenden offenen Seiten 14 der Dosierkavitäten 10 in Gewichtskraftrichtung nach unten, wobei die Dosierkavitäten 10 genau über je einer Zielkavität 13 zu liegen kommen. Hiervon ausgehend erfolgt nun eine Befüllung der Zielkavitäten 3, wozu das dosierte Pulver 2 aus den Dosierkavitäten 10 in die jeweils zugeordneten Zielkavitäten 3 überführt wird.

Zur Überführung des Pulvers 2 aus den Dosierkavitäten 10 in die Zielkavitäten 3 wird nun über den Druckkanal 16 ein entsprechender Druckverlauf aufgebracht. Dies kann ein kurzer Druckstoß mit positiver Druckdifferenz oder ein zwischen positiver und negativer Druckdifferenz oszillierender Luftdruck sein. Jedenfalls erfährt die Pulverfüllung durch das Filterelement 15 hindurch eine Druckänderung, was hier im weiteren Sinne als "Ausblasen" verstanden wird. Alternativ oder zusätzlich kann es zweckmäßig sein, einen mechanischen Impuls oder eine mechanische Schwingung auf das in den Dosierkavitäten 10 befindliche Pulver 2 aufzubringen. In allen Fällen wird eine Lösung des Pulvers 2 in den Dosierkavitäten 10 bewirkt, so dass es von dort in die Zielkavitäten 3 unter Einwirkung der Gewichtskraft fällt.

Im Anschluss an die Übergabe des Pulvers 2 an die Zielkavitäten 3 erfolgt eine weitere 90°-Drehung der genannten Anordnung. Das zuvor betrachtete Dosierorgan 8 erreicht nun die zweite Prüfstation 24 in der 270°-Position oder 3-Uhr-Position gemäß Fig. 2. Dort erfolgen optional Durchleuchtung, Kameraaufnahme und Auswertung analog zur ersten Prüfstation 23. Allerdings werden hierzu in der Auswerteeinrichtung 33 aus der von der Prüfkamera 32 aufgenommenen Lichtintensität Rückschlüsse darauf gezogen, ob das eingangs in der Dosierkavität 10 eingefüllte Pulver 2 ordnungsgemäß aus der Dosierkavität 10 ausgeblasen wurde. Bei negativem Prüfungsergebnis, wenn also aufgrund verringertem Lichtdurchlass Restmengen von Pulver 2 in einer oder mehreren Dosierkavitäten 10 festgestellt wurde, können geeignete Maßnahmen in die Wege geleitet werden.

Schließlich wird nun das Dosierorgan 8 mittels einer Schwenk- bzw. Rotationsbewegung um die Rotationsachse 17 entsprechend dem Pfeil 22 (Fig. 1) zurück zur Dosierstation 5 verfahren, wo der oben beschriebene Dosier- und Befüllzyklus erneut beginnt. Natürlich sind auch noch weitere Stationen beispielsweise in Form von Reinigungsstationen oder dergleichen im Rahmen der Erfindung einsetzbar.

Jedenfalls erfolgt die Prüfung in den Prüfstationen 23, 24 parallel zur Befüllung eines Dosierorgans 8 in der Dosierstation 5 und parallel zur gleichzeitigen Pulverübergabe eines anderen Dosierorgans 8 in der Füllstation 7, ohne die genannten Aktivitäten zu beeinträchtigen. Dadurch ist eine 100%-in-Prozess-Kontrolle ohne Verringerung der Taktzahl, wohl aber mit verbesserter Wiederholgenauigkeit und Zuverlässigkeit des Gesamtprozesses erreicht.

## Patentansprüche

1. Dosiereinrichtung (1) zum volumetrischen Dosieren eines insbesondere pharmazeutischen Pulvers (2) und zum Befüllen von mindestens einer Zielkavität (3) mit einer dosierten Pulvereinzelmenge (30) des Pulvers (2), wobei die Dosiereinrichtung (1) mindestens eine Dosierkavität (10) zur Bildung je einer dosierten Pulvereinzelmenge (30) umfasst, wobei die Dosierkavität (10) eine offene Seite (14) und gegenüberliegend dazu einen Boden aufweist, wobei der Boden der jeweiligen Dosierkavität (10) zumindest abschnittsweise durch ein luftdurchlässiges Filterelement (15) gebildet ist, wobei die Dosiereinrichtung (1) einen Druckkanal (16) aufweist, der mit der mindestens einen Dosierkavität (10) durch das Filterelement (15) hindurch druckübertragend verbunden ist,
**dadurch gekennzeichnet, dass** auf der der Dosierkavität (10) abgewandten Seite des Filterelementes (15) eine Prüflichtquelle (31) angeordnet ist, dass das Filterelement (15) bezüglich des von der Prüflichtquelle (31) ausgehenden Lichtes durchscheinend ausgestaltet ist, dass die Dosiereinrichtung (1) ferner eine Prüfkamera (32) sowie eine mit der Prüfkamera (32) verbundene Auswerteeinrichtung (33) umfasst, wobei die Prüfkamera (32) zumindest für einen Erfassungszeitraum auf die offene Seite (14) der Dosierkavität (10) gerichtet ist, und wobei die Auswerteeinrichtung (33) dazu ausgelegt ist, aus der von der Prüfkamera (32) aufgenommenen Lichtintensität des von der Prüflichtquelle (31) ausgehenden und durch das Filterelement (15) scheinenden Lichtes Rückschlüsse auf den Füllzustand der Dosierkavität (10) zu ziehen.

2. Dosiereinrichtung nach Anspruch 1,
**dadurch gekennzeichnet, dass** die Prüflichtquelle (31) in dem an das Filterelement (15) angrenzenden Mündungsbereich des Druckkanals (16) positioniert ist.

3. Dosiereinrichtung nach Anspruch 1 oder 2,
**dadurch gekennzeichnet, dass** die Prüflichtquelle (31) Lichtstreumittel (34) für eine gleichmäßige Ausleuchtung des Filterelementes (15) aufweist.

4. Dosiereinrichtung nach einem der Ansprüche 1 bis 3,
**dadurch gekennzeichnet, dass** die Dosiereinrichtung (1) mehrere Dosierkavitäten (10) aufweist, wobei die offenen Seiten (14) von mehreren und insbesondere von allen Dosierkavitäten (15) mittels der Prüfkamera (32) gemeinsam erfasst sind.

5. Dosiereinrichtung nach einem der Ansprüche 1 bis 4,
**dadurch gekennzeichnet, dass** die Dosiereinrichtung (1) eine Dosierstation (5), eine Füllstation (7), mindestens eine Prüfstation (23, 24) sowie ein bewegliches Dosierorgan (8) umfasst, wobei die Dosierkavität (10) und die Prüflichtquelle (31) im Dosierorgan (8) angeordnet sind, wobei die Prüfkamera (32) in der mindestens einen Prüfstation (23, 24) positioniert ist, und wobei das Dosierorgan (8) zyklisch zwischen der Dosierstation (5), der Füllstation (7) und der mindestens einen Prüfstation (23, 24) hin und her verfahrbar ist.

6. Verfahren zur Dosierung von insbesondere pharmazeutischem Pulver mittels einer Dosiereinrichtung (1) nach einem der Ansprüche 1 bis 5 und zum Befüllen von mindestens einer Zielkavität (3) mit einer dosierten Pulvereinzelmenge (30) des Pulvers (2), umfassend folgende Verfahrensschritte:
- Pulver (2) wird in die Dosierkavität (10) eingefüllt und später mittels durch den Druckkanal (16) und durch das Filterelement (15) hindurch aufgebrachten Gasdruck aus der Dosierkavität (10) ausgeblasen;
- Die Dosierkavität (10) wird mittels der Prüflichtquelle (31) durch das Filterelement (15) hindurch durchleuchtet;
- Die Prüfkamera (32) wird zumindest für einen Erfassungszeitraum auf die offene Seite (14) der Dosierkavität (10) gerichtet;
- Die Lichtintensität des von der Prüflichtquelle (31) ausgehenden und durch das Filterelement (15) scheinenden Lichtes wird von der Prüfkamera aufgenommen und in der Auswerteeinrichtung (33) ausgewertet;
- In der Auswerteeinrichtung (33) werden aus der von der Prüfkamera (32) aufgenommenen Lichtintensität Rückschlüsse auf den Füllzustand der Dosierkavität (10) gezogen.

7. Verfahren nach Anspruch 6,
**dadurch gekennzeichnet, dass** mittels der Prüfkamera (32) eine Verteilung der Lichtintensität über der Fläche der offenen Seite (14) der Dosierkavität (10) aufgenommen wird, und dass mittels einer Bildanalyse aus der aufgenommenen Verteilung der Lichtintensität Rückschlüsse auf den Füllzustand der Dosierkavität (10) gezogen werden.

8. Verfahren nach Anspruch 6 oder 7, umfassend folgende Verfahrensschritte:
- Pulver (2) wird in die Dosierkavität (10) eingefüllt;
- Die Dosierkavität (10) wird mittels der Prüflichtquelle (31) durch das Filterelement (15) hindurch durchleuchtet;
- Die Prüfkamera (32) wird zumindest für einen Erfassungszeitraum auf die offene Seite (14) der Dosierkavität (10) gerichtet;
- Die Lichtintensität des von der Prüflichtquelle (31) ausgehenden und durch das Filterelement (15) scheinenden Lichtes wird von der Prüfkamera aufgenommen und in der Auswerteeinrichtung (33) ausgewertet;
- In der Auswerteeinrichtung (33) werden aus der von der Prüfkamera (32) aufgenommenen Lichtintensität Rückschlüsse darauf gezogen, ob die Dosierkavität (10) ordnungsgemäß mit einer dosierten Pulvereinzelmenge (30) befüllt ist.
- Im Anschluss an die Aufnahme mittels der Prüfkamera (32) wird das Pulver (2) mittels durch den Druckkanal (16) und durch das Filterelement (15) hindurch aufgebrachten Gasdruck aus der Dosierkavität (10) ausgeblasen.

9. Verfahren nach einem der Ansprüche 6 bis 8, umfassend folgende Verfahrensschritte:
- Pulver (2) wird in die Dosierkavität (10) eingefüllt;
- Das in der Dosierkavität (10) befindliche Pulver (2) wird mittels durch den Druckkanal (16) und durch das Filterelement (15) hindurch aufgebrachten Gasdruck aus der Dosierkavität (10) in die Zielkavität (3) geblasen.
- Im Anschluss an das Ausblasen des Pulvers (2) wird die Dosierkavität (10) mittels der Prüflichtquelle (31) durch das Filterelement (15) hindurch durchleuchtet;
- Die Prüfkamera (32) wird zumindest für einen Erfassungszeitraum auf die offene Seite (14) der Dosierkavität (10) gerichtet;
- Die Lichtintensität des von der Prüflichtquelle (31) ausgehenden und durch das Filterelement (15) scheinenden Lichtes wird von der Prüfkamera aufgenommen und in der Auswerteeinrichtung (33) ausgewertet;
- In der Auswerteeinrichtung (33) werden aus der von der Prüfkamera (32) aufgenommenen Lichtintensität Rückschlüsse darauf gezogen, ob das eingangs in der Dosierkavität (10) eingefüllte Pulver (2) ordnungsgemäß aus der Dosierkavität (10) ausgeblasen wurde.

10. Verfahren nach einem der Ansprüche 6 bis 8,
**dadurch gekennzeichnet, dass** innerhalb eines Aufnahmeschrittes mehrere Lichtblitze von der Prüflichtquelle (31) ausgesandt werden.
